Europäisches Patentamt

European Patent Office

Office européen des brevets

(19)

(11) Publication number: **0 085 573**
**A2**

(12) # EUROPEAN PATENT APPLICATION

(21) Application number: **83300510.1**

(22) Date of filing: **01.02.83**

(51) Int. Cl.³: **C 12 N 5/02**

(30) Priority: **01.02.82 US 344673**

(43) Date of publication of application:
**10.08.83 Bulletin 83/32**

(84) Designated Contracting States:
**AT BE CH DE FR GB IT LI LU NL SE**

(71) Applicant: **NATIONAL FOUNDATION FOR CANCER RESEARCH, INC.**
**7316, Wisconsin Avenue Suite 851**
**Washington D.C. 20014(US)**

(72) Inventor: **Giaever, Ivar**
**2080 Van Antwerp Road**
**Schenectady New York 12309(US)**

(72) Inventor: **Keese, Charles Richard**
**Stony Brook Road**
**Scoharie New York 12157(US)**

(74) Representative: **Tubby, David George et al,**
**MARKS & CLERK 57-60 Lincoln's Inn Fields**
**London WC2A 3LS(GB)**

(54) **Interfacial growth of cells in tissue culture.**

(57) Cells are grown on or in droplets of a first liquid dispersed in a second liquid, where one of the liquids is sterile aqueous tissue culture medium and the other liquid is non-toxic to living cells and is relatively immiscible with water. An improved method employing a "stiffened" protein layer is also disclosed for cell culture in multi-phase liquid cell growth systems.

EP 0 085 573 A2

**0085573**

M&C FOLIO: 799P45569                    WANGDOC:0007i

## INTERFACIAL GROWTH OF CELLS IN TISSUE CULTURE

This invention relates to a tissue culture method in which cells are grown in vitro in appropriate tissue culture media.

Various cell culture techniques including general laboratory instructions, cell propagation on various supports, counting of cell populations, the preparation of media and reagents, may be found in the "Handbook of Cell and Organ Culture" by Merchant et al., Burgess Publishing Company, Minneapolis, 1964, Second Edition, and "Tissue Culture (Methods and Applications)" edited by P. F. Kruse, Jr. and M.F. Patterson, Jr., Academic Press, New York, 1973. In the commercial cultivation of cells, the cells grow as a monolayer on some sort of substrate, most commonly glass or a non-toxic plastics material, in contact with a tissue culture medium. Typically, the substrate may be the inner surface of a vessel containing the medium or may be the surface of small solid particles kept suspended in the medium. This latter mode of cell culture is also disclosed in "Microcarrier Culture of Vascular Endothelial Cells on Solid Plastic Beads" by P.F. Davies ["Experimental Cell Research" 134 (1981), pp. 367-376]. Several kinds of transformed cells will grow and divide in suspension in the tissue culture medium itself.

Still another method for cell and tissue culture which provides for the culture of cells at a liquid-liquid interface is described in the article by M. D. Rosenberg, "The Culture of Cells and Tissues at the Saline-Fluorocarbon Interface" in "Tissue Culture" (Proceedings of the Seminar held in Baroda under the auspices of the University Grants Commission and the Maharaja Sayajirao University of Baroda, India, January 21-28, 1965 edited by C.V. Ramakrishnan, Dr. W. Junk, publishers, the Hague, 1965, pp. 93-107). The cell culture system described by Rosenberg consists of a lower fluorocarbon liquid phase and an upper saline or nutrient liquid phase. The cells are grown in contact with the substantially planar interface between these two liquid phases. The use of silicone oils in place of the liquid fluorocarbons is also reported.

Further, it is reported in the article by N. G. Maroudas, "Chemical and Mechanical Requirements for Fibroblast Adhesion" ["Nature", Vol. 244 (August 10, 1973), pp. 353 and 354] that cells have been grown on the interface between a culture medium and droplets of silicone oil.

We have now discovered that cells can be grown on or in a large number of small droplets of a first liquid dispersed in a second liquid in the nature of an emulsion. One of these liquids is a sterile aqueous tissue culture medium and the other liquid, relatively immiscible with the tissue culture medium, is non-toxic to living cells. An emulsion is, typically, a heterogeneous system with at least one immiscible liquid dispersed in another in the form of droplets. The phase providing the droplets is the disperse, or internal, phase while the phase providing the matrix for the disperse phase is the continuous, or external, phase.

Thus the present invention consists in a process for growing cells in a tissue culture medium, in which cells are grown at the interface between a first liquid which is non-toxic to living cells and a second liquid, said second liquid being an aqueous sterile tissue culture medium, and said first and second liquids being relatively immiscible, characterized in that said first liquid is dispersed as a large number of protein-coated drops in said second liquid, to form an emulsion.

The process of the invention provides a greatly increased surface area (provided by the liquid droplets of the first liquid) on which cell growth may take place.

In practice, a volume of the non-toxic first liquid to be the disperse phase, typically a fluorocarbon, is placed in a container and a somewhat larger volume of sterile aqueous tissue culture medium (the second liquid) is poured into the same container. The emulsion is readily made by shaking the container so as to disperse the non-toxic liquid in the culture medium as many small droplets. The droplets preferably have sizes ranging from 50 micrometers to 400 micrometers and more preferably from 100 micrometers to 250 micrometers. Droplet density is preferably in the range of from 16,000 to 1,000,000 droplets per ml.

Thereafter, if there is a significant difference between the densities of the disperse and continuous phases, the system separates into two layers. In the case in which the non-toxic liquid selected is significantly heavier, the lower layer will comprise an emulsion rich in droplets of the non-toxic liquid dispersed in tissue culture medium and the upper layer will be primarily excess tissue culture medium. Next, the system is inoculated with the cells to be grown and, thereafter, is maintained under such conditions of temperature, pH, carbon dioxide concentration and oxygen tension as to facilitate cell growth. Prior to inoculation, each of the droplets will have acquired a coating of protein from the tissue culture medium, which typically contains a supplement of serum (e.g. 10% calf serum). After inoculation, the cells gradually settle through the liquid and reach the surface of the many small protein-coated droplets, where they adhere and grow as monolayers on the surfaces, receiving their nutrient from the culture medium bathing the protein-coated droplets. As the cells divide, they change from a flat configuration adhering tightly to the protein surface covering the droplets to a more rounded configuration, in which condition they can readily move to unoccupied protein-covered droplets in order to grow there. Assuming that the container is transparent, microscopic observations of the droplets with cells attached awash in the culture medium is greatly facilitated.

After the optimum period of growth for the cell/system combination (typically at least 24 hours), the harvesting of the cells may be accomplished by simply breaking the emulsion (i.e. demulsification). This breaking of the emulsion is readily accomplished by spinning or centrifuging the system, whereupon the disperse phase agglomerates; the two liquids separate creating a liquid-liquid interface; and, when the disperse phase is heavier than water, the cells go to the interface from which location they can be readily removed, as by the use of a pipette.

This harvesting procedure, in addition to the advantage of its simplicity, also has the advantage that the use of chelating agents or enzyme solutions, such as trypsin, pancreatin or collagenase is obviated. Not only is a process step thereby eliminated, but, in addition, the damage done to the surface to which they are adhered is avoided.

It should be understood that the present invention lies in the use of an emulsion to provide, at the interface between disperse and continuous phases, a large surface area on which cells may be grown. Accordingly, the nature of the cells to be cultivated is a matter of choice and is not a function of the present invention. For convenience, the invention is hereafter described primarily with reference to the cultivation of cells derived from animal tissue, but it will be appreciated that the invention is also applicable to the cultivation of a wide range of living cells, including microbial cells, whatever their origin or derivation.

The invention is of particular value for the cultivation of anchorage-dependent cells.

Both the Merchant et al. text and the Kruse, Jr. et al. text provide disclosure of the composition and preparation of many tissue culture media and various reagents employed in tissue culture. Many such media and media components are available commercially, as are many cell line starter cultures. Various liquids useful as the disperse phase are disclosed in the Rosenberg article.

As a variant of the above-described method of cell culture, this invention offers the advantage of using a "protein of choice" to coat the droplets and form a preselected base for cell growth. This capability increases the opportunity for optimization of cell growth. In this process, the non-toxic phase to be dispersed (e.g. a fluorocarbon liquid) is placed in a container, a solution of the protein of choice (e.g. fibronectin, collagen, gelatin) is added to the container and the container is shaken. Droplets of the disperse phase form with a coating of the protein of choice and settle. The supernatant protein solution is decanted; the tissue culture medium is added and then the system is inoculated with the cells to be grown. After cell growth has proceeded sufficiently, cell harvesting is accomplished as described above.

This invention has particular applicability to the cloning of specific cells. In this process a volume of tissue culture medium is inoculated with a quantity of the specific cells to be cloned. Using a pipette, individual droplets of the inoculated medium are suspended in a hydrophobic, non-toxic liquid, such as silicone oil, with which the medium will not mix. After a period of incubation, any of these droplets showing evidence under microscopic examination of sufficient cell growth within the droplet can be removed. Each medium droplet so removed will normally contain cells all grown from the same initial cell (i.e. clones).

In its best mode, this invention encompasses an improvement broadly applicable to the culture of cells in multi-phase liquid cell growth systems.  It has been found that both in the case of the stratified multi-liquid phase system of Rosenberg and in the case of the emulsion system described herein, cell growth is promoted by providing a "stiffened" protein layer at the interface(s) between the liquid phases in establishing the cell culture system.  Two modes for stiffening the protein layer on which cell growth is to occur are contemplated.  In the first mode a film of polymeric material, which carries a positive (i.e. basic) charge at physiological pH, underlies the protein cell growth layer at the interface(s).  In the second mode a cross-linking agent is permitted to react with the protein layer already at the interface(s).

One example of a useful basic (i.e. positively charged) polymer is poly-L-lysine.  The negatively charged protein layer, which subsequently deposits over the positively charged polymer film either by choice (i.e. "protein-of-choice") or inherently (i.e. from the serum content of the tissue culture medium), is thereby stiffened to provide a superior surface for cell attachment.  Increased adherence of cells to conventional solid tissue culture surfaces, when the surfaces are coated with polylysine or other basic polymers, is reported in the article "Stimulation of Clonal Growth of Normal Fibroblasts With Substrata Coated With Basic Polymers" by W.L. McKeehan et al. ["The Journal of Cell Biology", Vol. 71 (1976), pp. 727-734]. Growth of cells is also indicated as being improved on solid surfaces so treated.

In the case of cross-linking, after the protein layer has been deposited at the interface(s) between the liquid phases either as the protein-of-choice or inherently from the serum content of the tissue culture medium, a quantity of cross-linking agent, such as glutaraldehyde, is introduced, and, following the cross-linking reaction, the unreacted agent is removed from the system by rinsing. The use of such cross-linking agents to provide a cross-linked polyelectrolyte coat on aggregate material for the immobilization of microorganisms thereon is described in U.S. 4,287,305

Replenishment of the culture medium to the growing cells may be accomplished by gentle rotation of the container and, although not employed in the examples described herein, positive circulation (i.e. perfusion) of fresh medium to the growing cells is contemplated within this invention.

The invention is further illustrated by the following Examples, taken in conjunction with the accompanying drawings, in which:

Figure 1 is a schematic view in cross-section of an embodiment of this invention in which a container and its contents are employed for the growth cells over the surface of droplets of the disperse phase, and

Figure 2 is a schematic enlargement in cross-section showing one of the protein-coated droplets of the disperse phase bathed in the tissue culture medium.

## EXAMPLES

Cell spreading and growth on the surfaces of droplets of disperse-phase liquids in emulsions with tissue culture medium are demonstrated in the following Examples. Most of this work has been done with fluorocarbon liquids, because the droplets formed therefrom are readily stabilized, separate well upon breaking of the emulsion and are reusable after sterilization. These fluorocarbons are characterized by lack of toxicity, inertness, high density (about 1.9), immiscibility with water, low solubility for most other materials, thermal and chemical stability, low viscosity, transparency (i.e. for microscopic viewing) and hydrophobicity. The fluorocarbon oils used were FC-70 (primarily perfluoroamines) and FC-72 (primarily perfluorohexanes) marketed by the 3M Company, St. Paul, Minnesota, U.S.A. In addition, work was done with silicone oil (Dow Corning 704) and paraffin oil.

In addition to using 3T3-L1 ("normal" embryonic mouse fibroblast) and SV-T2 (virus-transformed mouse fibroblast) cell lines, normal human foreskin fibroblasts were used. All of these cells functioned as "anchorage dependent".

Although experiments are only reported using gelatin as the protein-of-choice, collagen and fibronectin can also be used.

In the preparation of the stabilized emulsion 1.0 ml of fluorocarbon liquid was dispersed in a larger volume (about 2.0 ml) of an aqueous solution of polylysine (2 mg/ml of poly-$\underline{L}$-lysine in 01N KOH) by rapid agitation in a vortex mixer until stabilized droplets in the approximate 100-400 micrometer diameter size range of fluorocarbon liquid dispersed in the aqueous solution were formed. The time/speed relationship to accomplish this for a given vortex mixer is routinely determinable. The molecular weight of the polylysine is not critical and satisfactory stabilized emulsions have been prepared using polylysine having molecular weights ranging from 3,000 to 240,000. Other polycations, such as spermadine, polyhistidine and polyarginine can be used. The KOH content of the aqueous polylysine solution serves to raise the pH of the solution considerably above the pK' value of the amino groups of the side chains in which condition (pH greater than

0085573

about 12) the polylysine is in a relatively uncharged state.  In this condition the polylysine provides much more complete coverage of the fluorocarbon droplets over the surface of which the polylysine in turn adsorbs as a monomolecular layer.

Once this stabilized emulsion has been formed, excess aqueous phase polylysine and the strong base (KOH) are removed from the emulsion.  The excess aqueous phase is first drawn off, after which several milliliters of 0.15 M sodium chloride in water were added and mixed gently with the emulsion.  The excess aqueous phase was again drawn off and this procedure was repeated several times.  This procedure was then repeated using complete culture medium containing animal serum (pH 7.4) in order to replace the sodium chloride solution with this culture medium and, thereby, to produce the desired stabilized emulsion in which fluorocarbon droplets are dispersed in tissue culture medium.  As a result of lowering the pH, the polylysine film on each droplet became positively charged, considerably enhancing the adsorption thereon of a layer of protein (negatively charged) from the animal serum in the culture medium.  This second monomolecular layer of serum proteins also contributed to the stability of the emulsion and the double layer provided a stiffer coating of protein for improved cell growth.

Although various standard tissue culture media having different quantities of serum can be used in the practice of this invention, the preferred culture medium is Dulbecco's modified Eagle's medium (DMEM) plus serum. Penicillin (100 units/ml) and streptomycin (100 micrograms/ml) were added to the DMEM as received.

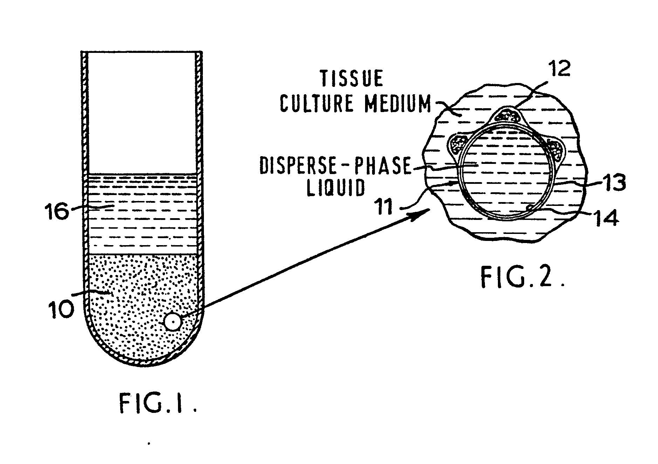

Inoculation of the emulsion with cells was accomplished by depositing the desired amount of cell suspension (about 250 x $10^3$ cells/ml in complete medium) by pipette over the emulsion. The cells settled down through the liquid to contact the protein-coated droplets of disperse phase liquid in the emulsion (the lower layer 10 of material in Figure 1). After sufficient incubation at 37°C in a suitable atmosphere (95% air, 5% $CO_2$, 100% relative humidity), the cells spread out over the stiffened protein layer on each droplet 11, grew and increased in numbers. As shown schematically in Figure 2, cells 12 attached to protein layer 13 grew over this outer surface area. As described herein, layer 14 represents a previously applied film of polymeric material, which carries a positive charge at physiological pH (i.e. a polycation). The emulsion stabilizing combination of layer 13 and layer 14 could, of course, be made up of a serum protein layer stiffened by a sufficient quantity of a cross-linking agent as described hereinabove.

If extensive cell growth is desired, the medium can be partially refurbished by periodically drawing off spent medium from layer 16 and replacing it with fresh sterile medium. This cell culture operation could, of course, be carried out in an appropriate apparatus such that fresh medium could be supplied by perfusion.

When cell culture was conducted in a standard microtiter plate with cylindrical (7 mm diameter) wells, 0.2 ml of protein-coated disperse phase droplets plus culture medium (i.e. emulsion) was pipetted into the well. Next, 0.1 ml of cell suspension (as above) in complete medium was pipetted over the emulsion. The remainder of the volume of the well was filled with complete culture medium to provide a total volume of about 0.4 ml in the well.

After each desired period of incubation, the extent of cell growth was readily determined by withdrawing a small amount of the inoculated emulsion and observing the droplets using phase contrast microscopy. Large increases (4 to 8-fold) in cell numbers have been observed with both SV-T2 and 3T3-L1 cells using droplets coated with single protein layers and droplets coated with stiffened protein layers. Ultimately, most of the droplets became covered with a layer of fibroblast growth over the protein layer.

By the process of this invention large quantities of undamaged cells grown on the much larger growth area provided by the protein-coated disperse phase droplets can very simply and efficiently be harvested. All that is required is to break the emulsion, as by centrifugation, whereupon the cells collect at the interface developed between the culture medium and the disperse phase (e.g. the fluorinated hydrocarbon). Centrifuge forces as high as 6000 times gravity have been used. Centrifugal forces of this magnitude normally break the emulsion in a matter of a few seconds. The cells, clearly visible at the phase boundary as an opaque mass, are readily removed by pipetting. The viability of these harvested cells is readily demonstrated by transferring some of these cells to a conventional cell culture substrate (e.g. polystyrene treated for cell culture) and supplying complete cell culture medium thereto.

Best results for cell growth were obtained using 3T3-L1 or SV-T2 cells and fluorocarbon liquids (FC-70, FC-72) as the disperse phase with the droplets (100-400 micrometers in diameter) coated with polylysine and these covered in turn with serum protein. Several other examples of cell growth systems and observations made are set forth in the following Table. The cell growth systems were prepared and operating conditions were set as described hereinabove. Droplet sizes are in the size range of about 100-400 micrometers. Unless otherwise indicated, all percentages are by volume. Observation time for Examples 1 and 2 were at 2 days and 4 days, while observation times for Examples 3 and 4 were 17 hours and 2 days. In the case of the paraffin oil emulsion, the droplets of paraffin oil in a matrix of cell culture medium floated on the balance of the aqueous culture medium.

TABLE

| EXAMPLE | DISPERSE PHASE DROPLETS | CULTURE MEDIUM | CELL INOCULANT | REMARKS |
|---|---|---|---|---|
| 1 | FC-70 fluorocarbon with polylysine – serum protein coat | 80% DMEM, 10% calf serum, 10% fetal calf serum | Newborn foreskin fibroblasts – normal human cells | Some cell attachment and spreading observed; cell growth probable |
| 2 | FC-70 fluorocarbon with polylysine – gelatin coat | same as for (1) | same as for (1) | same as for (1) |
| 3 | Silicone oil with polylysine – serum coat | 90% DMEM, 10% newborn calf serum | SV-T2 | Cell attachment, spreading and growth observed |
| 4 | Paraffin oil (s.g. less than 1.0) with polylysine – serum protein coat | same as for (3) | same as for (3) | Cell attachment and spreading observed |

17

0085573

CLAIMS:

1. A process for growing cells in a tissue culture medium, in which cells are grown at the interface between a first liquid which is non-toxic to living cells and a second liquid, said second liquid being an aqueous sterile tissue culture medium, and said first and second liquids being relatively immiscible, characterized in that said first liquid is dispersed as a large number of protein-coated drops in said second liquid, to form an emulsion.

2. A process according to claim 1, wherein the living cells are anchorage dependent.

3. A process according to claim 1 or claim 2, wherein the first liquid is significantly heavier than the second liquid.

4. A process according to any one of the preceding claims, wherein the protein coating on the droplets is in contact with a stiffening adjuvant.

5. A process according to claim 4, wherein the adjuvant is a polycation layer underlying and adhered to the protein coating, the polycation being applied to the droplets at a pH at which the polycation has a substantially neutral charge.

6. A process according to claim 5, wherein the polycation is polylysine, spermadine, polyhistidine or polyarginine.

7. A process according to claim 6, wherein the polycation is poly-L-lysine.

8. A process according to claim 4, wherein the adjuvant is a cross-linking agent.

9. A process according to claim 8, wherein the cross-linking agent is glutaraldehyde.

10. A process according to any one of the preceding claims, wherein the tissue culture medium contains serum.

11. A process according to any one of the preceding claims, wherein fresh sterile tissue culture medium is supplied to the emulsion during cultivation.

12. A process according to any one of the preceding claims, wherein the diameters of the droplets are in the range of from 100 micrometers to 250 micrometers.

13. A process according to any one of the preceding claims wherein the droplet density is in the range of from 16,000 to 1,000,000 droplets per ml of emulsion.

14. A process according to any one of the preceding claims, in which prior to addition of living cells said droplets are coated with a preselected protein.

15. A process accordingy to claim 14, in which said preselected protein is collagen or fibronectin.

16. A method of cloning cells in which:

(a) living cells are introduced into a sterile aqueous tissue culture medium;

(b) droplets of said medium bearing said cells are suspended in a non-toxic liquid immiscible therewith;

(c) said cells are cultivated;

(d) an individual droplet is thereafter removed; and

(e) cells are recovered from said individual droplet.

17. A process according to claim 16, wherein the non-toxic liquid is silicone oil.

18. A process for growing cells in a tissue culture medium, in which cells are grown at the interface between a first and a second liquid, one of said liquids being non-toxic to living cells and the other being an aqueous sterile tissue culture medium, said liquids being relatively immiscible, characterized in that said first liquid is dispersed as a large number of droplets in said second liquid, to form an emulsion.

19. In a system for growing cells in tissue culture medium wherein substantially planar interface area is established between a first fluid and a second fluid adjacent thereto, said first fluid being sterile tissue culture medium and said second fluid being both non-toxic to living cells and relatively immiscible with said first fluid and a protein layer upon which cell growth can occur extends along said interface area, the improvement wherein a stiffening adjuvant is in contact with said protein layer.

20. The improvement as defined in claim 19 wherein the fluids are liquids and the first fluid is located over the second liquid and is supported thereon.

21. In a system for growing cells in tissue culture medium wherein substantially planar interface area is established between a first liquid and a second liquid adjacent thereto, said first liquid being sterile tissue culture medium and said second liquid being both non-toxic to living cells and relatively immiscible with said first liquid and a layer of protein upon which cell growth can occur extends along said interface area, the improvement wherein instead of being substantially planar, said interface area consists of the aggregate of the surface areas of a large number of small protein-coated droplets of said second liquid dispersed in said first liquid to comprise an emulsion.

22. The improvement as defined in claim 21 wherein, in addition, a stiffening adjuvant is in contact with the protein coatings on the droplets.

23. The improvement as defined in claim 19 or 22 wherein the adjuvant is a polycation layer adhered to the protein coating.

24. The improvement as defined in claim 23 wherein the polycation is lysine, spermadine, polyhistidine or polyarginine.

25. The improvement as defined in claim 19 or 22 wherein the adjuvant is a cross-linking agent.

26. The improvement as defined in claim 25 wherein the cross-linking agent is glutaraldehyde.

27. The improvement as defined in claim 21 wherein the protein coating is of fibronectin.

28. The improvement as defined in claim 21 wherein the protein coating is of collagen.

29. The improvement as defined in claim 21 wherein the diameters of the droplets are in the range of from about 100 micrometers to about 250 micrometers.

30. The improvement as defined in claim 21 wherein the droplet density is in the range of from about 16,000 to about 1,000,000 droplets per cubic centimeter of emulsion.

TISSUE
CULTURE MEDIUM

DISPERSE-PHASE
LIQUID

16

10

11

12

13

14

FIG.1.

FIG.2.